# EUROPEAN PATENT SPECIFICATION

(11) **EP 0 404 938 B1**
(45) Date of publication and mention of the grant of the patent: **14.09.1994**
(21) Application number: 89903235.3
(22) Date of filing: 08.03.1989
(51) Int. Cl.: C07C 401/00

(54) **1 beta,7,8-TRIHYDROXYVITAMIN D 2, 1 beta,7,8-TRIHYDROXYVITAMIN D 3 OR THEIR DERIVATIVES, AND PROCESS FOR THEIR PREPARATION**
1 beta,7,8-TRIHYDROXYVITAMIN D 2, 1 beta,7,8-TRIHYDROXYVITAMIN D 3 UND IHRE DERIVATE UND VERFAHREN ZU IHRER HERSTELLUNG
1 beta,7,8-TRIHYDROXYVITAMINE D 2,1 beta,7,8-TRIHYDROXYVITAMINE D 3 OU LEURS DERIVES, ET PROCEDE DE PREPARATION

(30) Priority: 09.03.1988 JP 55342/88
(43) Date of publication of application: 02.01.1991
(73) Proprietor: DAIDO HOXAN INC., Chuo-ku Sapporo (JP)
(72) Inventor: TAHARA, Yuji, Hoxan Laboratory, Hoxan Corporation, Sapporo-shi, Hokkaido 003 (JP)
(74) Representative: Gibson, Stewart Harry
(86) International application number: JP8900249
(87) International publication number: WO8908640

(56) References cited:
- WO-A-86/07364
- JP-A-59 212 466
- JOURNAL OF ORGANIC CHEMISTRY, vol. 51, no. 8, 18th April 1986, pages 1269-1272, American Chemical Society; "J.L. MASCARENAS et al.: "Studies on the synthesis of side-chain hydroxylated metabolites of vitamin D. 3. Synthesis of 25-ketovitamin D3 and 25-hydroxyvitamin D3"

## Description

### FIELD OF THE INVENTION

This invention relates to 1β ,7,8-trihydroxy vitamin D₂, 1β ,7,8-trihydroxy vitamin D₃, their novel derivative compounds and a method of manufacturing these chemicals from 7,8-dihydroxy vitamin D₂, 7,8-dihydroxy vitamin D₃ or any of their derivatives.

### BACKGROUND OF THE INVENTION

The fact that vitamin D plays a vital role in formation of bones as it regulates absorption of calcium and reabsorption of bone inorganic substances by intestine has recently been made known by an extensive research on metabolites of bone (H. F. DeLuca et al., Ann. Rev. Biochem., vol. 52. p. 411, 1983).

It has also been known that some vitamin D₃ derivatives have the function of inducing cell differentiation, suggesting enormous potential applications of vitamin D in the future (T. Suda et al., Bone & Mineral Res./4. ed. W. A. Peck, Elsevier, Amsterdam, p. 1, 1986).

1α ,25-dihydroxy vitamin D₃ (N. Ikegawa et al., Organic Synthetic Chemistry, vol. 37, p. 755, 1979) and 1α - hydroxy vitamin D₃ (C. Kaneko, Organic Synthetic Chemistry, vol. 33, p 75, 1975) which are chemically analogous to each other and actively substantiate the above described effects have been attracting attention and are currently used as medicines for diseases such as chronic nephric insufficiency, while the latter has proved to be an excellent medicine for curing osteoporosis.

Under these circumstances, research has been actively conducted on synthetic preparation of derivatives of 1α -hydroxy vitamin D₃ which are regarded as indispensable for substantiating biogenic activities (B. Lythgoe, Chem. Soc. Rev., vol. 9, p. 449, 1980; R, Pardo et al., Bull. De La Soc. Chim. De Fr. p. 98, 1985).

Currently, the process of synthesis of derivatives of 1α -hydroxy vitamin D₃ typically starts with synthesis of 1α -hydroxylated steroid, which is converted to corresponding 1α -hydroxy-5,7 dienesterol and then to the intended vitamin D derivative by using known photochemical techniques. However, this known process comprises a number of steps, making the overall processes rather inefficient. (See above-cited C. Kaneko's paper.)

With a view to overcome these problems, a number of improved processes have recently been proposed, which can be categorized into the following three groups or groups (a), (b) and (c).

### Group (a)

1) direct hydroxylation of vitamin D₃ and related compounds at C(1); H. F. De Luca et al., J. Org. Chem., vol. 45, p. 3253, 1980
2) preparation of 1α -hydroxylated compounds; H. F. De Luca et at., Japanese Patent Application No. 54-555366
3) derivatives of cyclovitamin D; H. F. De Luca et al., Japanese Patent Application No. 57-206229
4) derivatives of 1α -hydroxycyclo vitamin D; H. F. De Luca et at., Japanese Patent Application No. 57-206230
5) derivatives of 1-oxocyclo vitamin D; H. F. De Luca et al., Japanese Patent Application No. 57-206231

In any of the group (a) processes, a vitamin D derivative is converted to a derivative of 3,5-cyclovitamin D, which is then combined by means of allylic oxidation at position C(1), said compound being further converted to a derivative of vitamin D.

### Group (b)

1) direct-positional or stereoselective α -hydroxylation of a (5E)-calciferol derivative; R. H. Hesse et al., J. Org. Chem., vol. 51, p. 1635, 1986
2) synthesis of 25-hydroxy vitamin D₃ and 1α ,25-dihydroxy vitamin D₃ from vitamin D₂; R. H. Hesse et al., J. Org. Chem., vol. 51, p. 4819, 1986
3) synthesis of MO-903, a metabolite of biogenetic vitamin D; M. J. Calverley, Tetrahedron, vol. 43, p. 4609, 1987

Any of the group (b) research papers proposes a process where vitamin D is temporarily combined by means of allylic oxidation at position C(1) to convert it to transvitamin D, which is then reconverted to vitamin.

### Group (c)

1) Synthesis of A-ring of 1S-hydroxykohlcalciferol; D. Desmaele et al., Tetrahedron Letters, vol. 16, p. 4941, 1985
2) Improved synthesis of an A-ring of 1α ,25-dihydroxy vitamin D; L. Castedo et al., Tetrahedron Letters, vol 28, p. 2099, 1987
3) Stereoselective synthesis of Lynthgoe A-ring aldehyde for synthesis of 1α -hydroxytachysterol and calciferol; E. G. Baggiolini et al., Tetrahedron Letters, vol. 28, p. 2095, 1987
4) simplified synthesis of Enyne A-ring of 1α - hydroxy vitamin D; W. H. Okamura et al., Tetrahedron Letters, vol. 28, p. 4947, 1987
5) stereoselective synthesis of 1α ,25-dihydroxykohlcalciferol and 1α ,25-dihydroxyergocalciferol; E. G. Baggiolini et al., J. Org. Chem. vol. 51, p. 3098, 1986
6) ergocalciferol derivatives; E. G. Baggiolini et al., Japanese Patent Application No. 59-52417
7) calciferol derivatives; E. G. Baggiolini et al., Japanese Patent Application No. 60-22091

Group (c) represents efforts to attain a totally synthetic process in which a chemical fragment that corresponds to an A-ring having a hydroxyl group at C(1) position is synthesized, to which fragments that correspond to a C-ring and a D-ring are combined to obtain the aimed compounds. However, such a process consists of a number of steps and hence does not provide a simple, economic and satisfactory method of manufacturing the compounds in practical applications. Moreover, compounds which are obtained by such a process do not necessarily exhibit satisfactory chemical effects.

It is therefore an object (the first object) of the present invention to provide novel and chemically useful 1β ,7,8-trihydroxy vitamin D₂, 1β ,7,8-trihydroxy vitamin D₃ and their derivatives.

It is another object (the second object) of the present invention to provide an industrially efficient and effective method of manufacturing above chemicals by directly combining vitamin D₂, vitamin D₃ or any of their derivatives with an oxygen function group at position C(1) through utilization of allylic oxidation as described later, a method which is completely different from any exisiting synthetic methods in terms of basic concept and practical applications.

The present invention provides a chemical compound of the formula (II) (a 1β, 7, 8-trihydroxy vitamin D₂, 1β, 7, 8-trihydroxy vitamin D₃ or a derivative thereof); 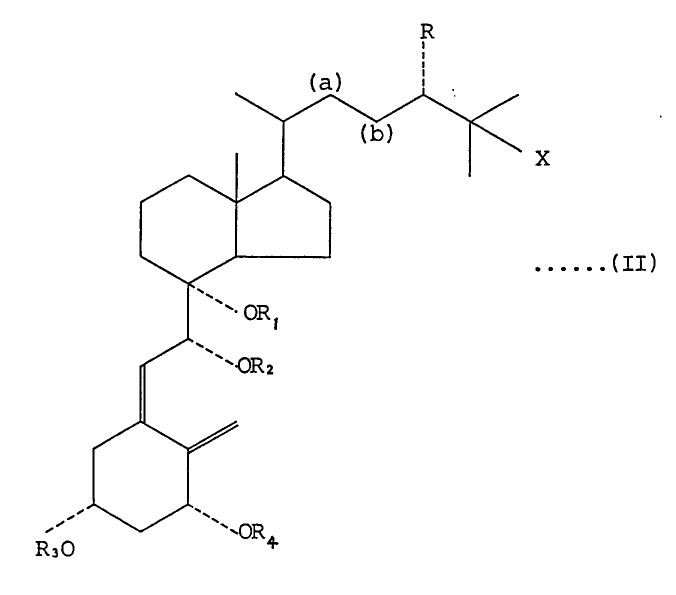
where R represents a hydrogen atom or a methyl group;
the carbon-carbon bond between atoms (a) and (b) in the side chain is either a single or double carbon-carbon bond;
each of R₁, R₂, R₃ and R₄ independently represents a hydrogen atom or a hydroxy protecting group; and
X represents a hydrogen atom or a hydroxy group.

There can easily be derived from compounds of formula (I) a fragment that corresponds to the A-ring to be used for combining it with fragments that correspond to a C-ring and a D-ring by cleaving its 7, 8 linkage by known techniques, in an oxidizing environment.

The present invention further comprises a method of producing a compound of formula (II) wherein 7, 8-dihydroxy vitamin D₃ or any of their derivatives of formula (I) 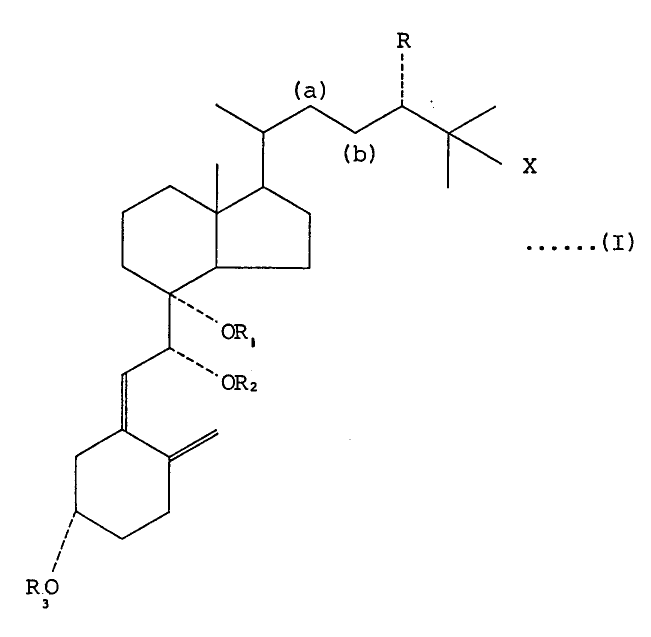
where R, R₁, R₂, R₃ and X are as defined above;
is reacted with a reagent comprising selenium dioxide in an inert solvent.

The method according to the invention can be used for industrial manufacture of chemical compounds of formula II by adding an oxygen functional group to the C1-position by means of allylic oxidation, which chemically advantageously affects the process of addition.

Compounds of formula (I) which can be used in the method according to the invention include the following known chemicals:
7, 8-dihydroxy-7, 8-dihydro vitamin D₃ (R₁=R₂=R₃=H), 3β - O-(t-butyldimethylsilyl)-7, 8-dihydroxy-7, 8-dihydro vitamin D₃ (R₁=R₂=H, R₃= -Si(Me)₂ t-Bu) [W.H. Okamura et al., J. Org. Chem., vol.48, p.1414, 1983], 7, 8-dihydroxy vitamin D₂ (R₁=R₂=R₃=H, R=Me, with a side chain) [Y. Wang et al., Acta. Chim. Sin., vol.24, P.126, 1958] and 7, 8, 25-trihydroxy vitamin D₃ derivatives (R₂=R₂=R₃=H or hydroxy protecting group, R=H, X=OR₄, and R₄=H or hydroxy protecting group, without a side chain) [H.F. Luca et al., Japanese patent application No. 59-93130].

A preferred solvent comprises either methylene chloride or acetonitrile. It is further preferred that the reagent further comprises t-butyl hydroperoxide.

The desired compound, of formula [II], can be separated from the reaction system conventionally. For example, after completion of the reaction, the reaction system may be washed with aqueous alkaline solution and thereafter with water, and then dried to remove the solvent; the residue may be subjected to colour chromatography.

Moreover, a compound expressed by formula [II] can be advantageously used to derive therefrom a fragment which corresponds to an A-ring which is to be combined with fragments which respectively correspond to C- and D-rings in the synthetic methods of forming vitamin D₃ derivatives described earlier with reference to group (c) by cleaving the 7,8 bond by means of oxidising agents following any known method. (See W. Wang et al., Acta, Chim. Sin., vol. 24, p. 126, 1958; and W. H. Okamura, ibid.)
The present invention will be described in greater detail by referring to a number of examples, in which "vitamin D" refers to "vitamin D₂" or "vitamin D₃" and the spectrum values are those of vitamin D₃.

### Example 1

### "Preparation of 7,8-dihydroxy-7,8-dihydro vitamin D 7,8-acetonide"

400mg of 7,8-dihydroxy-7,8-dihydro vitamin D and 0.5mℓ of 2,2-dimethoxypropane were dissolved in 5mℓ of dry methylene chloride. Stirring the solution at 0°C , camphor sulfonic acid was added thereto in a catalytic amount.

The solution was stirred for another two hours at room temperature and the reaction products were diluted with 20mℓ of methylene chloride and washed sequentially by a saturated aqueous solution of sodium hydrogencarbonate and water. Thereafter the residue was dried by sodium sulfate.

The residual product which was free from the solvent was then subjected to a process of silica gel column chromatography to obtain 7,8-dihydroxy-7,8-dihydro vitamin D 7,8-acetonide.

The above reaction is expressed by the following formula. 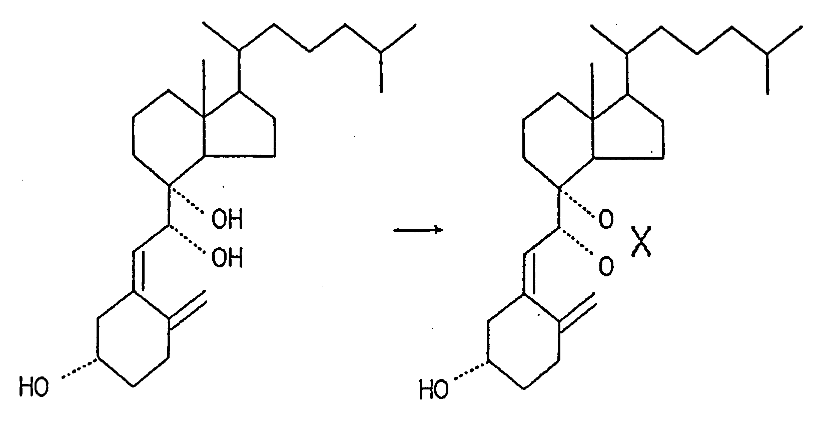
IR spectrum: ν max (CHCℓ₃ )cm⁻¹: 3400
MMR spectrum: (CCℓ₄)δ : 0.53(3H,S), 0.87(9H,d,J=6Hz), 1.19 (3H,S), 1.30(3H,S), 3.30-3.80(1H,m), 4.60 (1H,d,J=10Hz), 4.96 (1H,brs), 5.46(1H,d, J=10Hz)
mass spectrum: (FD)m/e; 459(M⁻+1), 458(M⁻), 443, 400, 383

### Example 2

### "Preparation of 3β -O-(t-butyldimethylsilyl)-7,8-dihydroxy-7,8-dihydro vitamin D 7,8-acetonide"

450mg of 7,8-dihydroxy-7,8-dihydro vitamin D 7,8-acetonide and 300mg of imidazole were dissolved in 30mℓ of dry dimethylformamide and then 300mg of t-butyldimethylsilyl chloride was added thereto while the solution was being stirred at 0°C .

The solution of the reaction products was stirred for another two hours at room temperature and then diluted by 100mℓ of methylene chloride. The diluted solution was washed sequentially by water, 10% hydrochloric acid, saturated aqueous solution of sodium hydrogencarbonate and water and the residue obtained by removing the solvent was subjected to a process of silca gel column chromatography [silica gel 6g, solvent; n-hexane-ethyl acetate (100: 1v/v)] to get 500mg of 3β -O-(t-butyldimethylsilyl)-7,8-dihydroxy-7,8-dihydro vitamin D 7,8-acetonide.

The above reaction is expressed by the formula shown below. 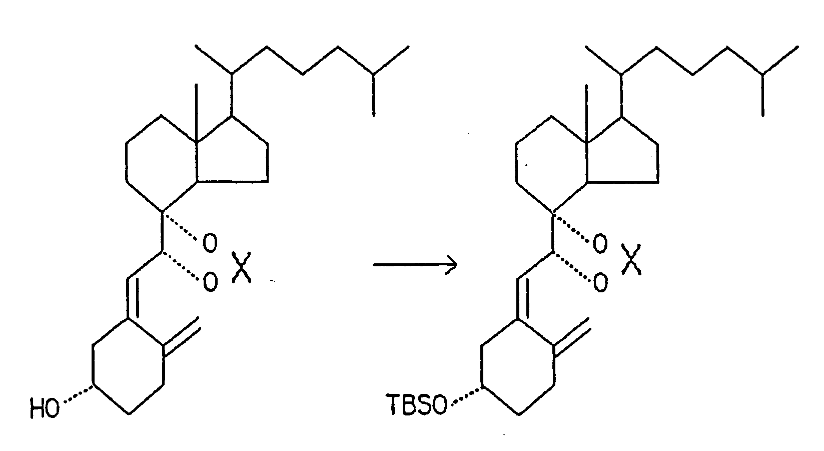
NMR spectrum: (CCℓ₄)δ : 0.06(6H,S), 0.53(3H,S), 0.85 (9H,d,J=6Hz), 0.93(9H,S), 1.20(3H,S), 1.30 (3H,S), 3.4-3.8(1H,m), 4.60(1H,d,J=10Hz), 5.00(2H,brs), 5.45(1H,d,J=10Hz)
mass spectrum: (FD)m/e; 573(M⁻+1), 572(M⁻, 557, 514, 457, 439

### Example 3

### "Preparation of 3β -O-(t-butyldimethylsilyl)-7,8-dihydroxy-7,8-dihydro-1β -hydroxy vitamin D 7,8-acetonide by oxidation of 3β -O-(t-butyldimethylsilyl)-7,8-dihydroxy-7,8-dihydro vitamin D 7,8-acetonide"

(a) 100mg of 3β -O-(t-butyldimethylsilyl)-7,8-dihydroxy-7,8-dihydro vitamin D 7,8-acetonide was dissolved in 20mℓ of dry methylene chloride, to which 70mg of selenium dioxide was added and the solution was heated for 20 hours for reflux while it was being stirred.
   After completion of the reaction, the solution was filtered by cerite and washed sequentially by 10% aqueous solution of sodium hydroxide and water. The product was dried by sodium sulfate and the residue obtained by removing the solvent was subjected to a process of silica gel column chromatography [silica gel 1g, solvent; n-hexane-ethyl acetate (100: 5v/v)] to get 55mg of 3β -O-(t-butyldimethylsilyl)-7,8-dihydroxy-7,8-dihydro-1β -hydroxy vitamin D 7,8-acetonide.
   IR spectrum: ν max (CHCℓ₃)cm-¹ : 3400
   NMR spectrum: (CCℓ₄)δ : 0.09(6H,S), 0.53(3H,S), 0.87(9H,d, J=6Hz), 0.93(9H,S), 1.20(3H,S), 1.30(3H,S), 3.60-4.10(lH,m), 4.60(lH,d,J=10Hz), 5.l6(lH, brs), 5.28(lH,brs), 5.58(lH,d,J=10Hz)
   mass spectrum (FD)m/e; 589(M⁻+1), 588(M⁻), 573, 530,513, 473, 455, 324
(b) 100mg of 3β -0-(t-butyldimethylsilyl)-7,8-dihydroxy-7,8-dihydro vitamin D 7,8-acetonide was dissolved in 20mℓ of dry methylene chloride, to which 10mg of selenium dioxide and 2mℓ of 3mol toluene solution of t-butylhydroperoxide were added while it was being heated and stirred for 20 hours for reflux.
   After completion of the reaction, the product was 50mg of 3β -O-(t-butyldimethylsilyl)-7,8-dihydroxy-7,8-dihydro-1β -hydroxy vitamin D 7,8-acetonide.
(c) 100mg of 3β -O-(t-butyldimethylsilyl)-7,8-dihydroxy-7,8-dihydro vitamin D 7,8-acetonide was dissolved in 20mℓ of dry methylene chloride, to which 10mg of selenium dioxide and 2mℓ of 3mol toluene solution of t-butylhydroperoxide as well as a catalytic amount of salicylic acid were added while it was being heated and stirred for 20 hours for reflux.
   After completion of the reaction, the product was treated similarly as in the case of (a) above to obtain 47mg of 3β -O-(t-butyldimethylsilyl)-7,8-dihydroxy-7,8-dihydro-1β -hydroxy vitamin D 7,8-acetonide.
(d) 100mg of 3β -O-(t-butyldimethylsilyl)-7,8-dihydroxy-7,8-dihydro vitamin D 7,8-acetonide was dissolved in 20mℓ of dry methylene chloride, to which 70mg of selenium dioxide and a catalytic amount of salicylic acid were added. The mixture was heated and stirred for 20 hours for reflux.

After completion of the reaction, the product was treated similarly as in the case of (a) above to obtain 45mg of 3β -O-(t-butyldimethylsilyl)-7,8-dihydroxy-7,8-dihydro-1β -hydroxy vitamin D 7,8-acetonide.

The reactions (a) through (d) above are expressed by the formula below.

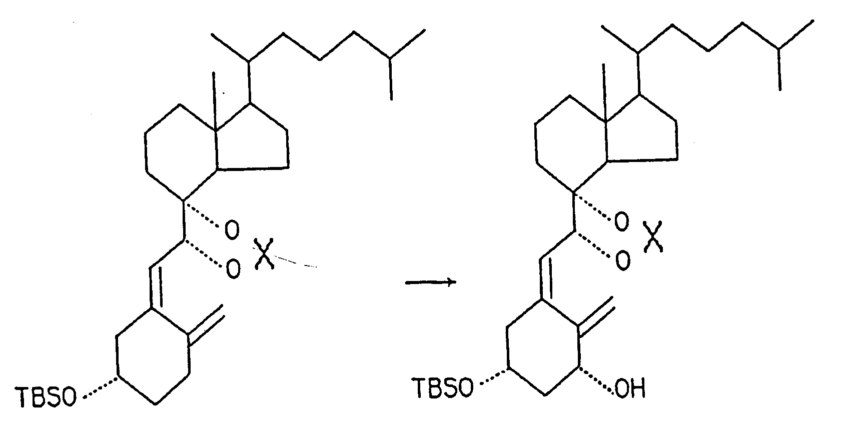

### Example 4

### "Preparation of 1β -acetoxy-3β -O-(t-butyldimethylsilyl)-7,8-dihydroxy-7,8-dihydro vitamin D 7,8-acetonide"

30mg of 3β -O-(t-butyldimethylsilyl)-7,8-dihydroxy-7, 8-dihydro-1β -hydroxy vitamin D 7,8-acetonide was dissolved in 1mℓ of dry pyridine, to which 10mg of acetyl-chloride was added while the solution was being stirred at 0°C.

The mixture of the reaction products was diluted by 30mℓ of methylene chloride and the diluted solution was stirred for four hours, which was then washed sequentially with water, 10% hydrochloric acid, saturated aqueous solution of sodium hydrogencarbonate and water and dried by sodium sulfate. Thereafter, the solvent was removed from the solution and the residue was subjected to a process of silica gel column chromatography [silica gel 1g, solvent; n-hexane-ethyl acetate (100: 1v/v)] to obtain 29mg of 1β - acetoxy-3β -O-(t-butyldimethylsilyl)-7,8-dihydroxy-7,8-dihydro vitamin D 7,8-acetonide.
IR spectrum: ν max (CHCℓ₄)δ : 0.10(6H,S), 0.56(3H,S), 0.88 9H,d,J=6Hz), 0.93(9H,S), 1.20(3H,S), 1.30(3H,S), 2.10(3H,S), 3.5-4.0(1H,m), 4.57(1H,d,J=10Hz), 4.80-5.20(1H,m), 4.95 (1H,brs), 5.60(1H,d,J=10Hz)
mass spectrum: (FD)m/e; 630(M⁻), 615, 573, 515, 366
The above reaction is expressed by the following formula.

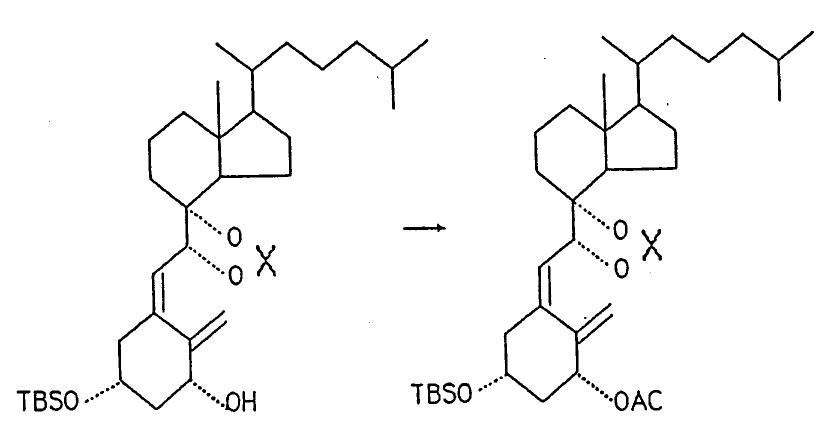

### Example 5

### "Preparation of 7,8-dihydroxy-7,8-dihydro-1β -hydroxy-vitamin D 7,8-acetonide"

(a) A catalytic amount of pyridinium-paratoluenesulfonate was added to 100mg of 3β -O-(t-butyldimethylsilyl)-7,8-dihydroxy-7,8-dihydro-1β -hydroxy vitamin D 7,8-acetonide dissolved in 30mℓ of methanol and the solution was heated and stirred for 10 hours for reflux.
   After completion of the reaction, the solvent was removed and the residue was dissolved in 30mℓ of chloroform. After washing the solution with water, it was dried by sodium sulfate and the solvent was removed. The product was subjected to a process of silica gel column chromatography (silica gel 1g, solvent; chloroform) to obtain 90mg of 7,8-dihydroxy-7,8-dihydro-1β -hydroxy vitamin D 7,8-acetonide.
   IR spectrum: ν max (CHCℓ₄)cm⁻¹ : 3400
   NMR spectrum: (CCℓ₄)δ : 0.5(3H,S), 0.88(9H,d,J=6Hz), 1.20 (3H,S), 1.33(3H,S), 3.50-4.30(2H,m), 4.70 (1H,d,J=10Hz), 5.05(1H,brs), 5.25(1H,brs) 5.70(1H,d,J=10Hz)
   mass spectrum: (FD)m/e; 475(M⁻+1), 474(M⁻), 459, 416, 399
(b) 100mg of 3β -O-acetyl-1β -acetoxy-7,8-dihydroxy-7,8-dihydro vitamin D 7,8-acetonide was dissolved with a catalytic amount of paratoluene sulfonic acid in 30mℓ of methanol and the solution was stirred for 10 hours.

After completion of the reaction, the catalyst was removed from the mixture of the reaction products the residue was dissolved in 50mℓ of methylene chloride and then washed sequentially with saturated aqueous solution of sodium hydrogencarbonate and water. Thereafter, the product was dried by sodium sulfate.

The dried product was treated similarly as in the case of (a) to obtain 91mg of 7,8-dihydroxy-7,8-dihydro-1β - hydroxy vitamin D 7,8-acetonide.

The reactions of Example 5 are expressed by the following formulae.

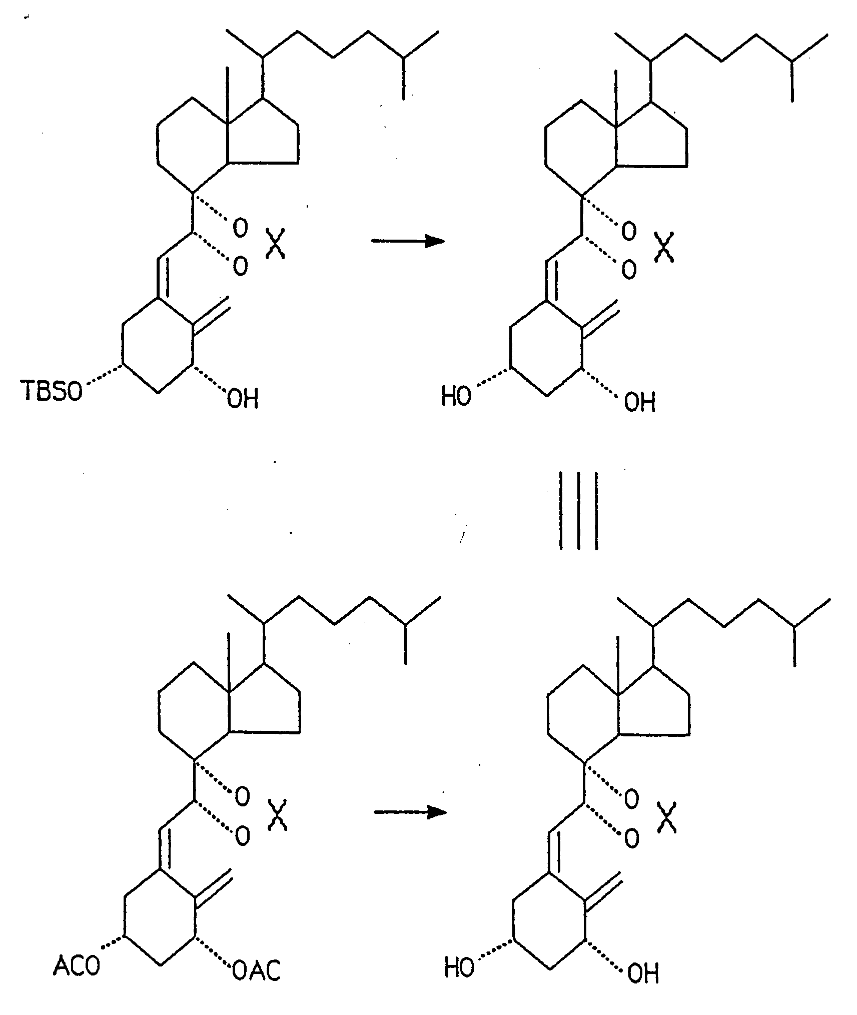

### Example 6

### "Preparation of 3β -O-acetyl-1β -acetoxy-7,8-dihydroxy-7,8-dihydro vitamin D 7,8-acetonide"

A 100mg of 7,8-dihydroxy-7,8-dihydro-1β -hydroxy vitamin D 7,8-acetonide was dissolved in 2mℓ of pyridine with a catalytic amount of 4-dimethylaminopyridine and 100mg of acetylchloride was added to the solution while it was being stirred at 0°C. The solution was further stirred for two hours at room temperature and then diluted with 50mℓ of methylene chloride. Afterwards, it was washed sequentially with water, 10% hydrochloric acid, saturated aqueous solution of sodium hydrogencarbonate and water and then dried by sodium sulfate.

The residue after removing the solvent was subjected to a process of silica gel column chromatography [silica gel 1g, solvent: n-hexane-ethyl acetate (100: 1v/v)] to obtain 102mg of 3β -O-acetyl-1β -acetoxy-7,8-dihydroxy-7,8-dihydro vitamin D 7,8-acetonide.
IR spectrum: ν max (CHCℓ₃)cm⁻¹ : 1730
NMR spectrum: (CCℓ₄)δ : 1.30(3H,S), 0.81(9H,d,J=6Hz), 1.20 (3H,S); 1.30(3H,S), 2.00(3H,S), 4.40-4.88 (1H,m), 4.90-5.30(1H,m), 4.60(1H,d,J=10Hz) 5.00(1H,brs), 5.18(1H,brs), 5.68(1H,d,J=10Hz)
mass spectrum: (FD)m/e; 558(M⁻), 543, 501, 294, 264
The reaction of Example 6 is expressed by the following formula.

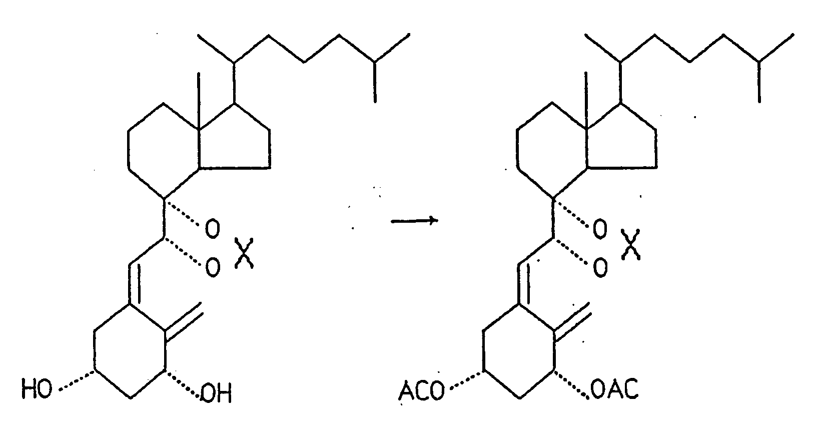

### Example 7

### "3β -O-(t-butyldimethylsilyl)-7,8-dihydroxy-7,8-dihydro-1β - hydroxy vitamin D"

(a) 100mg of 3β -O-(t-butyldimethylsilyl)-7,8-dihydroxy-7,8-dihydro vitamin D was dissolved in 30mℓ of dry methylene chloride, to which 100mg of selenium dioxide was added. The solution was then heated and stirred for three days for reflux.
   After completion of the reaction, the mixture of the reaction products was washed sequentially with 10% aqueous solution of sodium hydroxide and water and dried with sodium sulfate.
   After removing the solvent, the residue was subjected to a process of silica gel column chromatography [silica gel 1g, n-hexane-ethyl acetate (10: 1v/v)] to obtain 57mg of 3β -O-(t-butyldimethylsilyl)-7,8-dihydroxy-7,8-dihydro-1β -hydroxy vitamin D.
   IR spectrum: ν max (CHCℓ₃)cm⁻¹ : 3400
   NMR spectrum: (CCl₄)δ : 0.13(6H,S), 0.80(3H,S), 0.90(9H,d, J=6Hz), 0.95(9H,S), 3.90-4.30(2H,m), 4.75 (1H,d,J=10Hz), 4.80(1H,brs), 5.60(1H,brs) 5.83(1H,d,J=10Hz)
   mass spectrum 8FD)m/e; 549(M⁻+1), 548(M⁻), 531, 513, 473, 455, 283, 265
(b) 100mg of 3β -O-(t-butyldimethylsilyl)-7,8-dihydroxy-7,8-dihydro vitamin D was dissolved in 20mℓ of dry acetonitrile, to which 60mg of selenium dioxide was added. The solution was then heated and stirred for one hour for reflux.
   After completion of the reaction, the solvent was removed from the mixture of the reaction products and the residue was dissolved in 50mℓ. The solution was washed sequentially with 10% aqueous solution of sodium hydroxide and water and dried with sodium sulfate.
   The product was treated similarly as in the case of (a) above to obtain 12mg of 3β -O-(t-butyldimethylsilyl)-7,8-dihydroxy-7,8-dihydro-1β -hydroxy vitamin D.
(c) The same amount of the reagents and that of the solvent as in (b) were made to react at room temperature for two days and then the product was treated in a similar manner to obtain 10mg of 3β -O-(t-butyldimethylsilyl)-7,8-dihydroxy-7,8-dihydro-1β -hydroxy vitamin D from 100mg of 3β -O-(5-butyldimethylsilyl)-7,8-dihydroxy-7,8-dihydro-vitamin D.
(d) A mixture containing the same amount of the reagents and that of the solvent as in (a) and a drop of water were heated for three days for reflux. The reaction product was treated similarly as in the case of (a) to obtain 55mg of 3β -O-(t-butyldimethylsilyl)-7,8-dihydroxy-7,8-dihydro-1β - hydroxy vitamin D out of 100mg of 3β -O-(t-butyldimethylsilyl)-7,8-dihydroxy-7,8-dihydro vitamin D.
(e) The same amount of the reagents and that of the solvent as in (a) were heated with 3mℓ of acetonitrile for 13 hours for reflux. The reaction product was treated similarly as in the case of (a) to obtain 60mg of 3β -O-(t-butyldimethylsilyl)-7,8-dihydroxy-7,8-dihydro-1β -hydroxy vitamin D out of 100mg of 3β -O-(t-butyldimethylsilyl)-7,8-dihydroxy-7,8-dihydro vitamin D.

The reactions of Example 7 are expressed by the following formula.

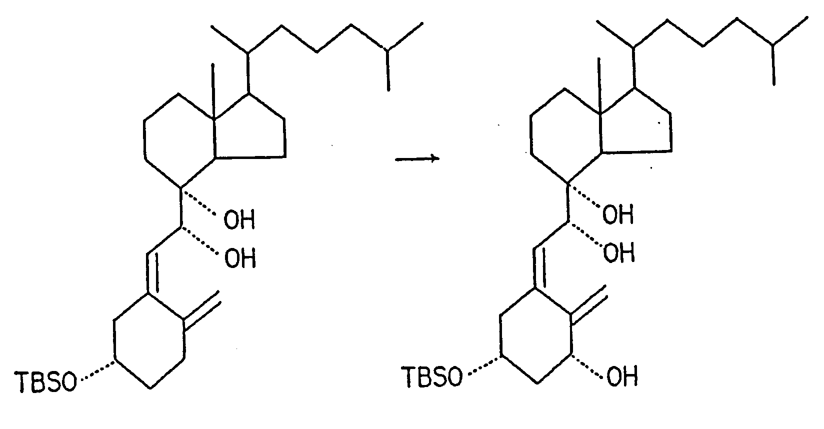

### Example 8

### "Preparation of 3β -O-(t-butyldimethylsilyl)-7,8-dihydroxy-7,8-dihydro-1β -hydroxy vitamin D 7,8-acetonide by acetonidation of 3β -O-(t-butyldimethylsilyl)-7,8-dihydroxy-7,8-dihydro-1β -hydroxy vitamin D"

10mg of 3β -O-(t-butyldimethylsilyl)-7,8-dihydroxy-7,8-dihydro-1β -hydroxy vitamin D and 0.1mℓ of 2,2-dimethoxypropane were dissolved in 2mℓ of methylene chloride and the solution was stirred at 0°C with a catalytic amount of camphor sulfonic acid. The solution was further stirred at room temperature for two hours and then diluted with 10mℓ of methylene chloride. The diluted solution was washed sequentially with saturated aqueous solution of sodium hydrogencarbonate and water and dried by sodium sulfate.

After removing the solvent, the residue was subjected to a process of silica gel column chromatography [silica gel 0.5g, solvent; n-hexane-ethyl acetate (100: 1v/v)] to obtain 10mg of 3β -O-(t-butyldimethylsilyl)-7,8-dihydroxy-7.8-dihydro-1β -hydroxy vitamin D 7,8-acetonide.

The product which had been obtained by the chemical equation as shown below was compared with the product of Example 3 by means of the IR and NMR spectrum technique; the two products were chemically completely identical.

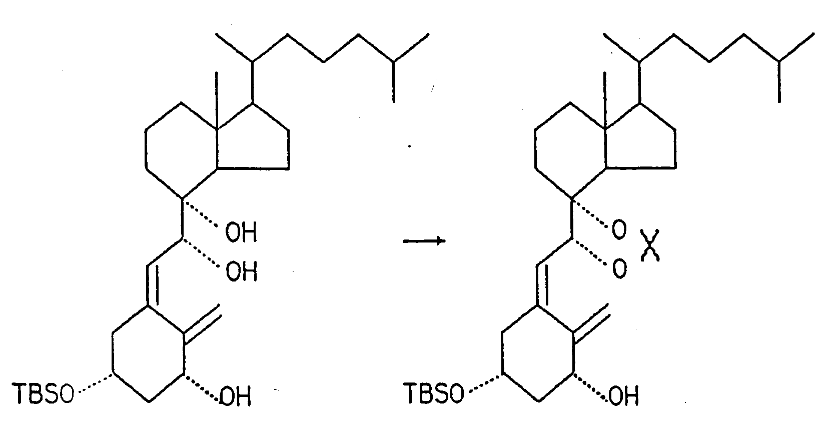

### Example 9

### "Preparation of 3β -O-(t-butyldimethylsilyl)-7,8-dihydroxy-7,8-dihydro-1β -t-butyldimethylsilyloxy vitamin D"

92mg of 3β -O-(t-butyldimethylsilyl)-7,8-dihydroxy-7,8-dihydro-1β -hydroxy vitamin D, 50mg of imidazole and a catalytic amount of 4-dimethylaninopyridine were dissolved in 2mℓ of dry dimethylformamide and 50mg of t-butyldimethylsilyl was added to the solution while it was being stirred.

After completion of the reaction, the solution of the reaction product was stirred for 24 hours at room temperature and was diluted with 50mℓ of methylene chloride. The diluted solution was then washed sequentially with water, 10% hydrochloric acid, saturated aqueous solution of sodium hydrogencarbonate and water and later dried by sodium sulfate.

After removing the solvent, the residue was subjected to a process of silica gel column chromatography [silica gel 1g, solvent; n-hexane-ethyl acetate (100: 5v/v)] to obtain 100mg of 3β -O-(t-butyldimethylsilyl)-7,8-dihydroxy-7,8-dihydro-1β -t-butylmethylsilyl vitamin D.
NMR spectrum: (CCℓ₄)δ : 0.07(12H,S), 0.77(3H,S), 0.86 (9H,d,J=6Hz), 0.90(9H,S), 0.96(9H,S), 3.30-4.10(2H,m), 4.70(1H,d,J=10Hz), 5.00(1H,brs), 5.30(1H,brs), 5.56(1H,d,J=10Hz)
mass spectrum (FD)m/e; 663(M⁻+1), 662(M⁻), 645, 627, 587, 530, 397, 339, 265
The reaction of Example 9 is expressed by the following formula.

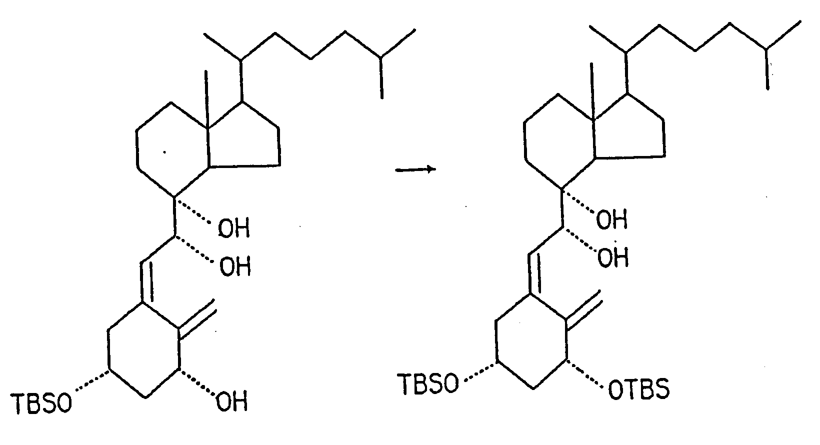

### Example 10

### "Preparation of 1β -acetoxy · 3β -O-(t-butyldimethylsilyl)-7,8-dihydroxy-7,8-dihydro vitamin D"

120mg of 3β -O-(t-butyldimethylsilyl)-7.8-dihydroxy-7,8-dihydro-1β -hydroxy vitamin D, 2mℓ of pyridine and a catalytic amount of 4-dimethylaminopyridine were dissolved in 5mℓ of methylene chloride and then 100mg of acetylchloride was added to the solution while it was being stirred at 0°C.

The solution of the reaction product was kept being stirred for 2 hours at room temperature and thereafter diluted by 30mℓ of methylene chloride. The diluted solution was washed sequentially with water, 10% hydrochloric acid, saturated aqueous solution of sodium hydrogencarbonate and water and dried by sodium sulfate.

After removing the solvent, the residue was subjected to a process of silica gel column chromatography [silica gel 0.5g, solvent; n-hexane-ethyl acetate (100: 5v/v)] to obtain 121g of 1β -acetoxy · 3β -O-(t-butyldimethylsilyl)-7,8-dihydroxy-7,8-dihydro vitamin D.
IR spectrum: ν max (CCHℓ₃)cm-¹ : 3350, 1740
NMR spectrum: (CCℓ₄)δ : 0.10(6H,S), 0.75(3H,S), 0.90(9h,d, J=6Hz), 0.95(9H,S), 2.10(3H,S), 3.35-4.00 (1H,m), 4.70(1H,d,J=10Hz), 4.60-5.00(1H,m), 5.05(2Hbrs), 5.63(1H,d,J=10Hz)
mass spectrum: (FD)m/e; 573(M⁻ -17), 530, 515, 489, 473, 325, 265
The reaction of Example 10 is expressed by the following formula.

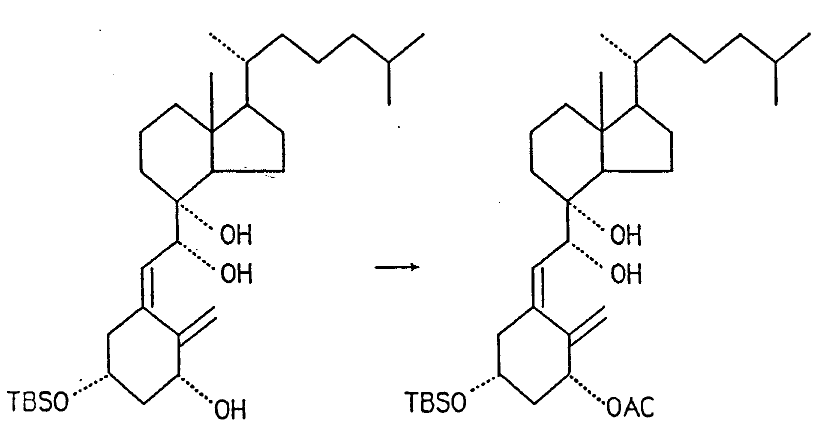

### Example 11

### "Preparation of 3β -O-(t-butyldimethylsilyl)-1β ,7-diacetoxy-8-hydroxy-7,8-dihydro vitamin D"

A 100mg of 3β -O-(t-butyldimethylsilyl)-7,8-dihydroxy-7,8-dihydro-1β -hydroxy vitamin D and a catalytic amount of 4-dimethylaminopyridine were dissolved in 2mℓ of pyridine and 100mg of acetic anhydride was further added to the solution at 0°C.

The solution of the reaction product was stirred for two hours at room temperature and diluted with 50mℓ of methylene chloride. Thereafter, the diluted solution was washed sequentially with water, 10% hydrochloric acid, saturated aqueous solution of sodium hydrogencarbonate and then dried by sodium sulfate.

After removing the solvent, the residue was subjected to a process of silica gel column chromatography [silica gel 0.5g, solvent; n-hexane-ethyl acetate (100: 3v/v)] to obtain 110mg of 3β -O-(t-butyldimethylsilyl)-1β ,7-diacetoxy-8-hydroxy-7,8-dihydro vitamin D.
IR spectrum: ν max (CHCℓ₄)cm⁻¹ : 3570, 1730
NMR spectrum: (CCℓ₄)δ : 0.13(6H,S), 0.70(3H,S), 0.90(9H, d,J=10Hz), 0.93(9H,S), 2.03(3H,S), 2.14 (3H,S), 3.50-4.10(1H,m), 4.80-5.00(1H,m) 5.40(1H,d,J=10Hz), 5.66(1H,brs), 5.98(1H, d,10Hz)
mass spectrum (FD)m/e; 632(M⁻), 615, 573, 555, 367, 265
The reaction of Example 11 is expressed by the following formula.

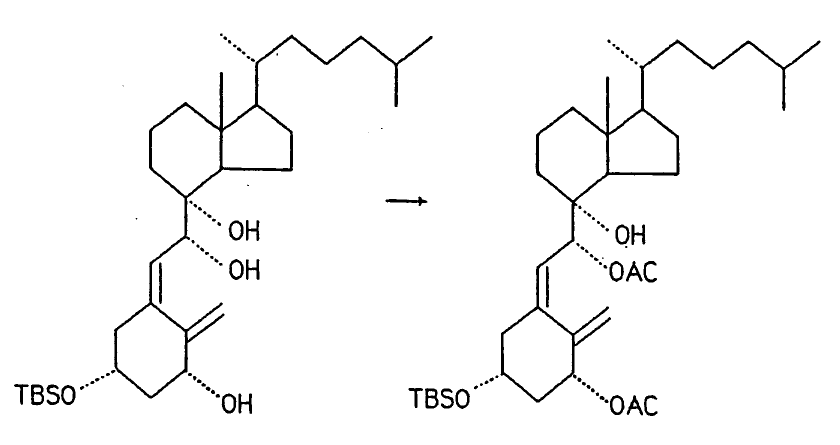

### Example 12

### "Preparation of 1β -acetoxy-3β -O-(t-butylmethylsilyl)-7,8-O-ethoxymethylene-7,8-dihydroxy-7,8-dihydro vitamin D"

10mℓ toluene solution containing 100mg of 1β - acetoxy-3β -O-(t-butylmethylsilyl)-7,8-dihydroxy-7,8-dihydro vitamin D, 100mg of ethylorthoformate and a catalytic amount of camphor sulfonic acid were heated for 30 hours for reflux while it was being stirred in a Dean stark apparatus.

After removing the solvent, the residue was subjected to a process of silica gel chromatography [silica gel 0.5g, solvent; n-hexane-ethyl acetate (100: 1v/v)] to obtain 100mg of 1β -acetoxy-3β -O-(t-butyldimethylsilyl)-7,8-O-ethoxy-methylene-7,8-dihydroxy-7,8-dihydro vitamin D.
IR spectrum: ν max (CHCℓ₃ )cm⁻¹: 1730
NMR spectrum: (CCℓ₄)δ : 0.10(6(H,S), 0.50(3H,S), 0.87(9H, d,J=6Hz), 0.90(9H,S), 2.13(3H,S), 3.50(2H, q,J=10Hz), 3.50-4.00(1H,m), 4.66(1H,d, J=10Hz), 5.00-5.20(2H,m), 5.48(1H,S), 5.70 (1H,d,J=10Hz)
mass spectrum: (FD)m/e; 647(M⁻+1), 646(M⁻), 600, 572, 556, 515, 501
The reaction of Example 12 is expressed by the following formula.

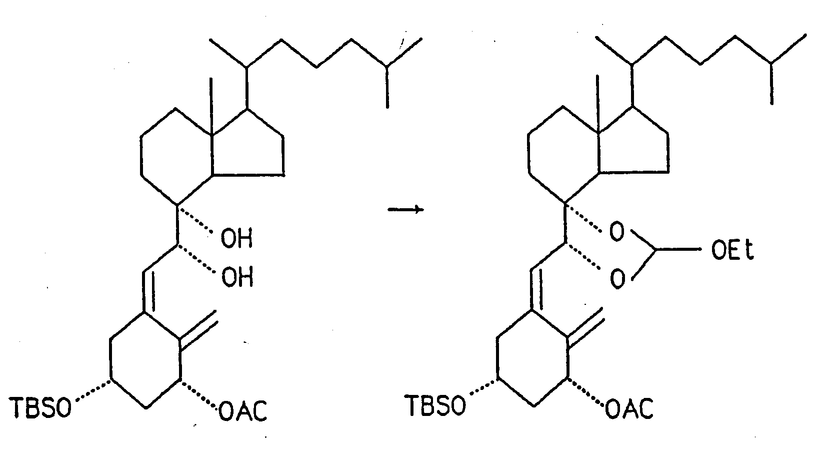

### POTENTIAL INDUSTRIAL APPLICATIONS

Compounds according to the present invention are novel substances generally expressed by formula [II] above. These substances typically include 1β ,7,8-trihydroxy vitamin D₂, 1β ,7,8,-trihydroxy vitamin D₃ and their derivatives that have many potential industrial applications as they can play a vital role in formation of bones because they can regulate absorption of calcium and reabsorption of inorganic substances by intestine. Moreover, they can be effective for inducing cell differentiation and also for curing such diseases as chronic nephric insufficiency and osteoporosis.

If compared with any known similar methods, the method according to the invention is industrially more efficient and effective for manufacturing novel chemical compounds generally expressed by formula [II] above as any of such compounds can be prepared by directly combining a compound expressed by formula [I] above with an oxygen function group at position C(1) through utilization of allylic oxidation.

## Claims

1. A chemical compound of the formula (II) 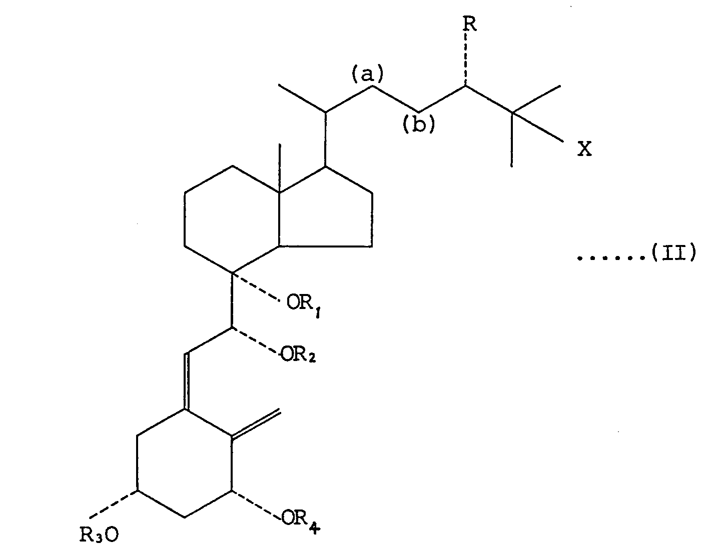 where R represents a hydrogen atom or a methyl group;
the carbon-carbon bond between atoms (a) and (b) in the side chain is either a single or double carbon-carbon bond;
each of R₁, R₂, R₃ and R₄ independently represents a hydrogen atom or a hydroxy protecting group; and
X represents a hydrogen atom or a hydroxy group.

2. A method of manufacturing a compound according to claim 1, wherein a compound of the formula (I) 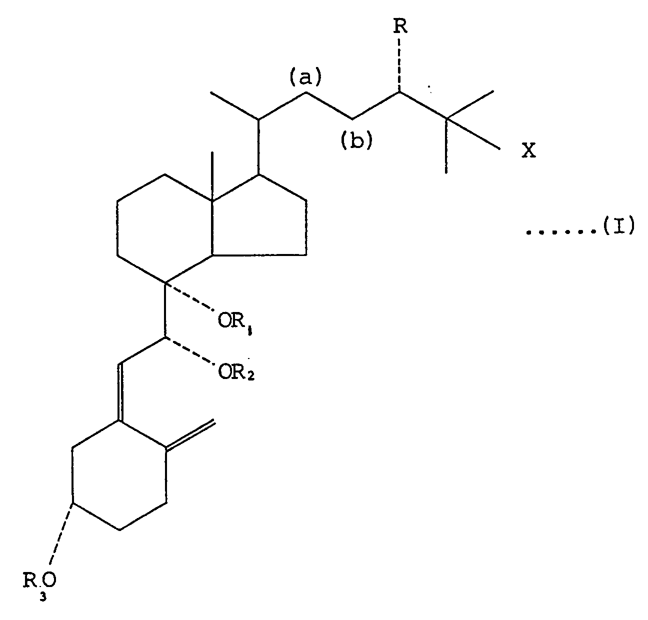 where R represents a hydrogen atom or a methyl group;
the carbon-carbon bond between atoms (a) and (b) in the side chain is either a single or double carbon-carbon bond;
each of R₁, R₂ and R₃ independently represents a hydrogen atom or a hydroxy protecting group; and
X represents a hydrogen atom or a hydroxy group;
is reacted with a reagent comprising selenium dioxide in an inert solvent.

3. A method according to claim 2, wherein said solvent comprises either methylene chloride or acetonitrile.

4. A method according to claim 2 or 3, wherein said reagent further comprises t-butylhydroperoxide.

## Patentansprüche

1. Chemische Verbindung der Formel (II) 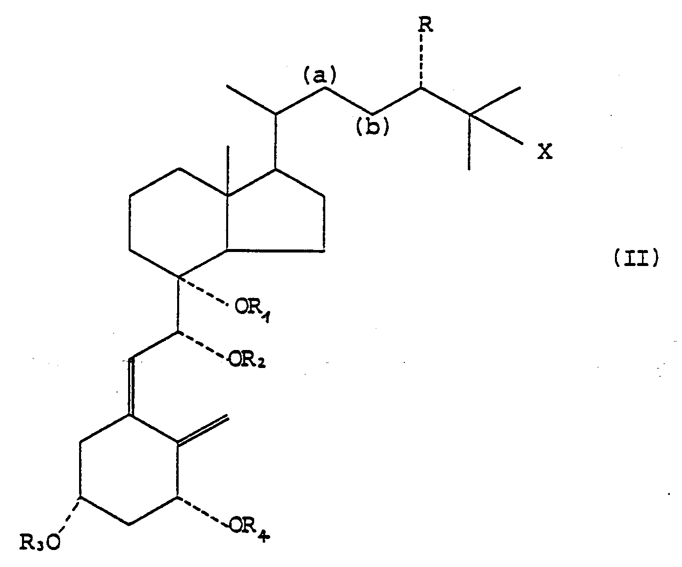 worin R ein Wasserstoffatom oder eine Methylgruppe, die Kohlenstoff-Kohlenstoff-Bindung zwischen den Atomen (a) und (b) in der Seitenkette entweder eine Kohlenstoff-Kohlenstoff-Einfachbindung oder eine Kohlenstoff-Kohlenstoff-Doppelbindung, jeder der Reste R₁,R₂,R₃ und R₄ unabhängig ein Wasserstoffatom oder eine Hydroxylschutzgruppe sowie X ein Wasserstoffatom oder eine Hydroxylgruppe bedeuten.

2. Verfahren zum Herstellen einer Verbindung nach Anspruch 1, worin eine Verbindung der Formel (I) 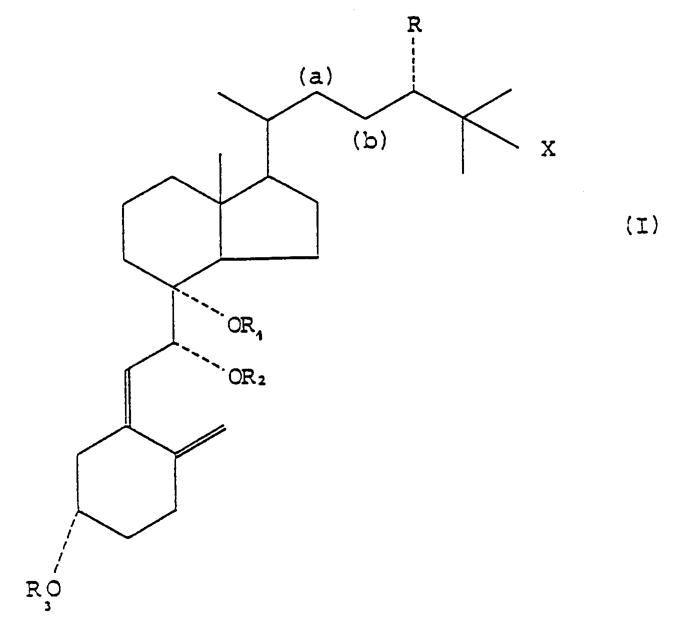 worin R ein Wasserstoffatom oder eine Methylgruppe, die Kohlenstoff-Kohlenstoff-Bindung zwischen den Atomen (a) und (b) in der Seitenkette entweder eine Kohlenstoff-Kohlenstoff-Einfachbindung oder eine Kohlenstoff-Kohlenstoff-Doppelbindung, jeder der Reste R₁,R₂ and R₃ unabhängig ein Wasserstoffatom oder eine Hydroxylschutzgruppe und X ein Wasserstoffatom oder eine Hydroxylgruppe bedeuten, in einem inerten Lösungsmittel mit einem Selendioxid enthaltenden Reagens umgesetzt wird.

3. Verfahren nach Anspruch 2, worin das Lösungsmittel entweder Methylenchlorid oder Acetonitril enthält.

4. Verfahren nach Anspruch 2 oder 3, worin das Reagens ferner tert.-Butylhydroperoxid enthält.

## Revendications

1. Produit chimique de formule (II) 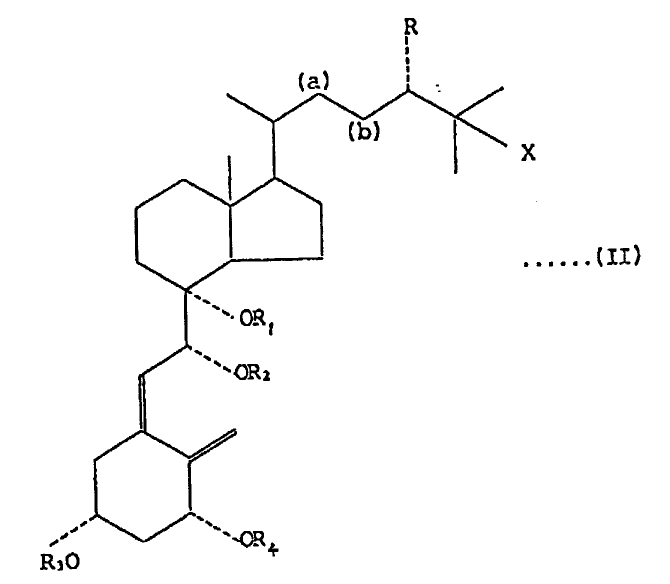 dans laquelle R représente un atome d'hydrogène où un groupe méthyle ; la liaison carbone-carbone entre les atomes (a) et (b) de la chaîne latérale étant une liaison simple ou double; R1, R2, R3, R4 -représentant chacun indépendemment un atome d,hydrogène ou un groupe protecteur hydroxyde ; et
X représentant un atome d'hydrogène ou un groupe hydroxyde.

2. Procédé de fabrication d'un produit selon la revendication 1 dans lequel on fait réagir un produit de formule (I) 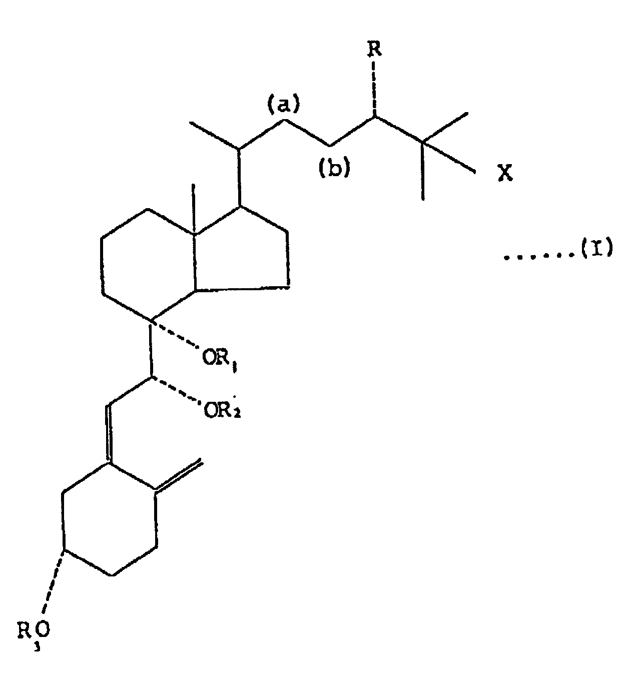 dans laquelle R représente un atome d'hydrogène ou un groupe méthyle;
la liasion carbone-carbone entre les atomes (a) et (b) de la chaîne latérale étant une liasion simple ou double;
R1, R2, R3, R4 représentant chacun indépendemment un atome d'hydrogène ou un groupe protecteur hydroxyde; et
X représentant un atome d'hydrogène ou un groupe hydroxyde;
avec un réactif comprenant du dioxyde de selenium en solution dans Un solvant inerte.

3. Procédé selon la revendication 2 dans lequel ledit solvant comprend du chlorure de méthylène ou de l'acétonitrile.

4. Procédé selon les revendications 2 ou 3, dans lequel ledit réactif comprend de plus du t-butylhydroperoxide.
